# EUROPEAN PATENT APPLICATION

(11) **EP 4 478 041 A1**
(43) Date of publication of application: **18.12.2024**
(21) Application number: 23179153.4
(22) Date of filing: 14.06.2023
(51) Int. Cl.: G01N 27/48, B01L 3/00, G01N 33/18

(54) **PORTABLE SYSTEM FOR MEASURING THE CONCENTRATION OF ELECTROCHEMICALLY ACTIVE SUBSTANCES IN WATER WITH ELECTROCHEMICAL TECHNIQUES**

(71) Applicant: Microx S.r.l., 38068 Rovereto (IT)
(72) Inventor: PRAVATO, Luca, 35035 Mestrino (IT); IANNONE, Eugenio, 20127 Milano (IT)
(74) Representative: Giraldi, Elisa

(57) **Abstract**

A portable system for measuring the concentration of electrochemically active substances in water with electrochemical techniques comprises:
- a device (10,13) for collecting a sample (C) of water to be analyzed,
- a device (10,14) adapted to mix the collected water sample (C) with a saline solution (B),
- a measuring cartridge (20) for analyzing the sample and saline solution mixture, wherein said cartridge is provided with at least one sensor (24),
- a reader (30) adapted to read the data produced by the sensor (24) of said measuring cartridge (20), and
- a data processing unit provided with a user interface for communication with an operator and a network interface for communication with a remote server or other type of remote apparatus acting as a user of the information, wherein said data processing unit converts the data from the analog form in which they are produced by the sensor (24) of the cartridge (20) into a digital format suitable for being further processed.

## Description

### FIELD OF THE INVENTION

The present invention relates to the field of measuring the concentration of electrochemically active substances in water. In particular, the present invention relates to a portable system for measuring the concentration of electrochemically active substances in water with electrochemical techniques.

### PRIOR ART

In the prior art it is known to carry out the analysis of pollutants in water by collecting a sample of water from the mass to be analyzed by means of a syringe system and transporting it to an analysis laboratory.

This procedure is the main cause of the fact that from the moment of the collection to the moment in which the results are available, a period of typically a few days passes. Such a duration is not caused by the time needed to perform the analysis itself, but by the transport, laboratory storage, and result communication times.

Normally, in fact, the analysis laboratories process the samples in the order of arrival and have both incoming and outgoing queues.

The results being not immediately available is a strong limitation of the effectiveness and efficiency of the analyses if a polluting event or a malfunction of some water treatment system is underway. Not being able to intervene immediately can cause problems for the health of both humans and crops or animals.

To obviate this problem, portable analysis systems exist, which however are not sufficiently accurate and reliable. In general, such systems give only qualitative results, i.e., they are capable of detecting the presence of a certain substance, but not the amount thereof, which makes the test unhelpful in most cases.

Once in the laboratory, in the vast majority of cases the analysis systems used are of two types.

The absolute reference in terms of sensitivity and accuracy consists of mass spectrometers, which are expensive and complex machines requiring highly qualified staff in order to be used. Alternatively, over-the-counter electrochemical systems are available, which are not as accurate as the mass spectrometers, but amply sufficient for the application. Highly qualified staff are needed in this case as well.

Both the cost of the machines and reagents and the need for specialized staff affect the costs of laboratory analyses, which to date are of the order of a few hundred euros for a standard analysis which quantifies a dozen pollutants.

Both the time needed and the costs limit the number of tests which are performed, generating the need to reduce costs and accelerate timeframes, possibly until obtaining results in real time.

### SUMMARY OF THE INVENTION

The object of the invention has been achieved by a system as defined in claim 1.

A portable system for measuring the concentration of electrochemically active substances in water comprises a device for collecting a sample of water to be analyzed, a device adapted to mix the sample of water collected with a saline solution, a measuring cartridge for analyzing the sample and saline solution mixture, in which the cartridge is provided with at least one sensor, a reader adapted to read the data produced by the sensor of the measuring cartridge, and a data processing unit provided with a user interface for communication with an operator and a network interface for communication with a remote server or with other devices connected to the network, in which the data processing unit converts the data from the analog form in which they are produced by the sensor of the cartridge into a digital format suitable for being further processed.

The device for collecting a water sample is a syringe comprising a main containment body provided with a plunger and a connection spout for the coupling and connection with a collection tip and a filtering tip.

The collection tip comprises an aspiration portion and a connection element. Preferably, the aspiration portion is hollow and allows the passage of liquids and is curved to facilitate the sample extraction operation.

In various embodiments, the device adapted to mix the collected sample of water with a saline solution is included in the filtering tip.

Preferably the filtering tip comprises an introduction portion, a connection element and a containment portion containing the saline solution and the connection element is sealed by a breaking closure.

In embodiments the containment portion and the introduction portion are two distinct parts inserted into each other so that they can rotate with respect to each other, in which a filtering part is included between the containment portion and the introduction portion and in which the containment portion is sealed by a membrane (M).

The connection between the connection spout of the syringe and the connection element of the collection tip or of the filtering tip occurs by interlocking or screwing to avoid accidental removal.

In some embodiments, the measuring cartridge is a microfluidic cartridge and contains at least one sensor which physically measures the concentration of electrochemically active substances.

The cartridge comprises a funnel inlet or recess which is connected to a microfluidic reservoir through a connection duct. Electrodes forming the sensor are arranged on the upper face of the microfluidic reservoir.

Preferably the sensor comprises a working electrode, a reference electrode and a counter electrode.

The cartridge inlet and the electrical contacts of the sensor are protected by a tear-off label.

In alternative embodiments the device adapted to mix the collected sample of water with a saline solution is a microfluidic mixer of the diffusive or chaotic type included in the microfluidic measuring cartridge.

The reader for reading from the cartridge contains all the electronics required to process the analog signal from the cartridge and present the data in a comprehensible manner.

Alternatively, the reader for reading from the cartridge uses the electronics of a smart device to process the analog signal from the cartridge and for the interaction of the reader with the operator.

The method for measuring the concentration of electrochemically active substances in water with electrochemical techniques comprises the steps of:
a. switching on the reader;
b. removing the label which protects the electrical contacts and inserting the cartridge into the reader;
c. extracting the syringe from the package with the collection tip already assembled on the syringe;
d. immersing the collection tip in water;
e. aspirating a sample of water up to the point marked on the syringe;
f. overturning the syringe and removing the collection tip;
g. assembling the filtering tip; during assembly, the closure label of the containment portion containing the measurement saline solution is broken, and the mixing between the buffer and the water sample begins;
h. after assembling the filtering tip on the syringe, rotating the introduction portion with respect to the containment portion so as to tear the membrane to allow the passage of liquid from the containment portion to the tip of the introduction portion,
i. removing the protection label of the cartridge thus opening the inlet thereof;
l. bringing the end opening of the filtering tip in contact with the cartridge inlet at the recess;
m. injecting the syringe water into the cartridge;
n. pressing the start button on the reader;
o. placing the syringe with the assembled filtering tip in the package for later recycling;
p reading the result on the screen after the set time;
q. extracting the cartridge;
r. switching off the reader; and
s. placing the used cartridge in the package for disposal or recycling.

### BRIEF DESCRIPTION OF THE DRAWINGS

Reference is made later in this description to the figures in the accompanying drawings, in which:
Figure 1 shows an example of a test cartridge and syringe for the insertion of the water sample to be analyzed,
Figure 2A shows a stand-alone reader, integrating the user interfaces and electronics needed for processing, and Figure 2B shows a reader connected to a smartphone,
Figure 3 shows the syringe for collecting the water sample to be analyzed with the two collection and filtering tips,
Figure 4 shows some details of the structure of the filtering tip,
Figure 5 shows some sections of the cartridge for electrochemical measurement,
Figure 6 shows an example of commercial micro-electrodes of circular shape,
Figure 7 shows an example of cartridge assembly, and
Figure 8 shows a simultaneous electrochemical measurement of the concentration of six heavy metals in a standard aqueous solution using the "DEP-On-Go" system known in the literature.

The parts according to the present description have been depicted in the drawings, where appropriate, with conventional symbols, showing only those specific details which are pertinent to understanding the embodiments of the present invention, so as not to highlight details which will be immediately apparent to those skilled in the art, with reference to the description provided below.

### DETAILED DESCRIPTION OF THE INVENTION

The solution of the present invention is now described with the aid of the drawings.

The system in hand, by way of example, is capable of measuring the concentration in water of a set of heavy metals, which are pollutants with a strong electrochemical response, dictated by the specific application. These concentrations will be measured simultaneously or in subsequent tests on the same water flow.

The main technological challenge is the need to measure very small concentrations, especially if the quality of drinking water is being certified. Different regulations exist for drinking water and waste water: the maximum levels prescribed by different regulations vary with the emergence of new rules which respond to the need to limit different types of adverse effects.

The solution proposed herein has been studied to overcome the limitations and disadvantages of the known solutions.

As for the structure of the system, the main requirements are as follows:
- the system must be portable, thus have a small size and weight;
- the system must be operable by a single person without the need for a second operator present;
- the operations of collecting the sample and loading the measuring system must be simple so that they can be performed by a basic operator and the presence of a specialized operator is not required;
- for reasons related to operator safety, the loading of the sample must not require contact between the operator and the water flow to be analyzed;
- for security issues of the acquired data, the system must have a data locking function, i.e., once acquired they cannot be modified by the operator;
- the system must have a local interface allowing the operator to read the data just measured, so that an alert can be started immediately if the thresholds are exceeded and a critical condition is detected;
- the system must allow, when required, connection to a local computer for subsequent operations such as downloading the acquired data for further analysis or checks;
- the system must be connectable to the mobile network and, if required, capable of transferring data acquired therethrough to a remote server; and
- the transfer of data to the network must occur in a secure manner without the possibility of them being tampered with during the transfer.

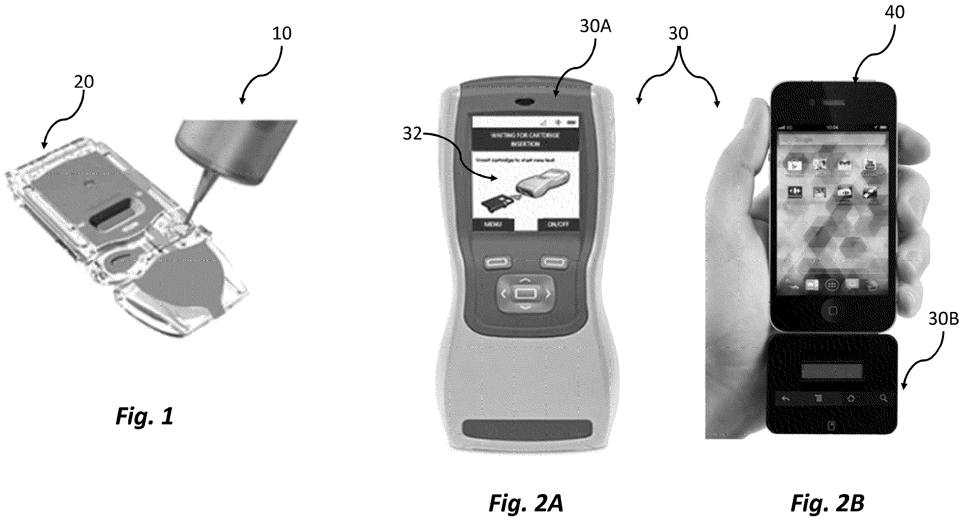

The architecture of the system is diagrammatically shown in Figures 1 and 2. The system in the basic version thereof comprises:
- a filtering syringe 10 by means of which a sample of water C to be analyzed is collected from the flow;
- a cartridge 20, in which the water sample C to be analyzed is inserted by means of the aforesaid filtering syringe 10 used for collection; and
- a reader 30, in which the cartridge 20 is inserted, for the analysis of the water sample C to be analyzed.

Preferably, the measuring cartridge 20 is a microfluidic cartridge, but it can also be a cartridge in which the dimensions of the ducts are also larger (of the order of cm), whereby the flow can no longer be considered microfluidic.

It is the task of the reader 30 to collect and perform a first processing of the raw data, converting them from the analog form in which they are produced by one or more sensors 26, present in the cartridge 20, into a digital format suitable for being further processed.

Figure 1 diagrammatically shows the measuring cartridge 20 and the syringe 10 for collecting the water sample C to be analyzed.

Figure 2 shows two possible embodiments of the reader 30 of the analysis system of the present solution. In particular, Figure 2A shows a stand-alone reader 30a, integrating the user interfaces and electronics required for processing. Vice versa, a reader 30B connected to a smartphone 40 is shown in Figure 2B. This second version is cheaper and takes advantage of the intelligence, computing power and user interfaces of the smartphone 40 for the interaction of the reader 30B with the operator.

Figure 2A shows a reader 30A including therein the digital data processing unit, a user interface, and all interfaces allowing remote communication of data through the network or dedicated links, such as Bluetooth.

Alternatively the reader 30B can only be an adaptive interface with an external device, such as a smartphone or tablet 40, as shown in Figure 2B. In this case, the reader 30B contains the electronics required to convert the native signal into a digital form compatible with a standard interface and all the processing is performed in the external device 40 in which suitable programs must be loaded, such as an App to allow communication with the reader 30B.

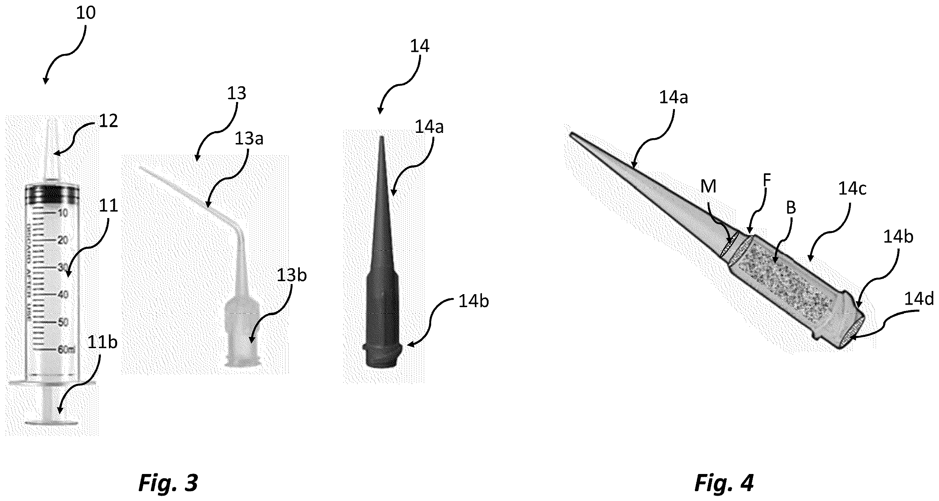

As shown in Figure 3, the filtering syringe 10 has several purposes:
- collecting the sample of water C to be analyzed from the main flow;
- combining the water sample C with a controlled amount of a saline buffer B required to perform the electrochemical measurement; and
- removing, by means of a filter F, the particles which could interfere with the measurement.

The syringe 10 comprises a main containment body 11 which has the shape of a hollow cylinder, provided with a plunger 11b, in which the main body 11 ends with a connection spout 12 for the coupling or connection with a collection tip 13 and a filtering tip 14.

The collection tip 13 and the filtering tip 14 comprise an aspiration or introduction portion, respectively 13a and 14a, and a connection element 13b and 14b. The aspiration portion 13a and the introduction portion 14a are hollow and allow the passage of liquids such as the water to be analyzed.

The connection between the connection spout 12 and the connection element 13b and 14b of the tips can occur by interlocking due to the pressure and friction between the parts (with dimensional interference) or screwing, which locks the connection element 13b and 14b on the connection spout to prevent the accidental removal thereof.

In some embodiments the collection tip 13 has the aspiration portion 13a curved to facilitate the collection.

The filtering tip 14 includes a containment portion 14c, containing the electrochemical buffer, between the introduction portion 14a and the connection element 14b. The filtering part or filter F is positioned between the containment portion 14c and the introduction portion 14a. The connection element 14b is sealed by a breaking closure 14d, such as a metal film, so as to preserve the electrochemical buffer.

A functional manner of closing the filtering tip 14 in the lower part consists of dividing such a tip into two parts 14a and 14c, as shown in Figure 4, inserted into each other with a bayonet mechanism so that they can rotate with respect to each other.

The section containing the buffer B is sealed below by a membrane M, while the introduction portion 14a internally has teeth (not shown in Figure 4). When the introduction portion 14a is pressed onto the containment portion 14c just before joining the two parts, the teeth enter into the membrane M and tear it. Afterwards, the membrane M is then completely detached from the rotation of the containment portion 14c with respect to the introduction portion 14a so as to allow the insertion of the liquid into the syringe in the microfluidic chip.

The main containment body 11 of the syringe 10 is calibrated so that the collected water sample C has a controlled amount and, when it is mixed with the measuring saline solution, the salt concentration can be controlled with good precision.

In general, the electrochemical measurement does not depend extremely quickly on the concentration of the saline buffer B; therefore, even possible errors in the mixing percentage do not generate significant errors in the next measurement. As for the buffer B, the electrochemical measurement requires that the solvent in which the molecule of which the concentration is to be measured is dissolved has a high and known saline content. In particular, the measurement depends on which and how many salts are present in the solution.

Since the collected water sample C will generally have a low saline content, it will be necessary to add a controlled amount of salts thereto, so that the initial saline content is negligible with respect to the added salts, thus allowing the measurement results to be correctly interpreted.

As for the particles P, they are particles of micrometric size, typically between 1 µm and a few tenths of a micron. Currently, the main source of such particles P is the microplastics present in waste water as well as, although to a lesser extent, in drinking water.

However, many other types of micrometer-sized particles P can be present. These particles P must be removed from the water sample C before injecting it into the measuring cartridge 20, to prevent these particles P from depositing on the sensors 26, altering the measurement itself.

Since the particles P must be removed without making a selection thereof according to the composition thereof, it is possible to use a very simple filtering agent, for example a filter paper F suitably inserted into the initial part of the syringe, i.e., into the filtering tip 14 thereof.

In particular, the filter F is positioned in the filtering tip 14 so that the particles P, collected together with the water sample C to be analyzed with the collection tip 13, can be retained in the filtering tip 14.

During the procedure of collecting the water sample C, the collection tip 13 is used to fill the syringe 10. At the end of the collection, the collection tip 13 is removed and the filtering tip 14 is inserted.

During the insertion of the filtering tip 14 on the syringe 10, the seal created by the breaking closure 14d is opened due to the connection spout 12 of the syringe 10. At this point, the syringe 10 with the filtering tip 14 is ready to perform the analysis of the sample C by means of the cartridge 20.

The most effective and least expensive solution for the withdrawal operation is the use of a plastic syringe 10 with a first collection tip 13 coupled in the syringe by means of a screw or bayonet coupling. After collecting the water sample C to be analyzed, the syringe 10 is overturned so as not to pour the collected water sample C and the collection tip 13, used to collect the sample, is detached and specially disposed of.

A second tip 14 containing a high-concentration saline solution known as buffer B to be mixed with the water sample C to be analyzed is then inserted on the syringe 10.

The filtering tip 14 incorporates a filter F, for example in cellulose, so that the water, mixed with the buffer B, is filtered immediately before the injection thereof into the cartridge 20.

The mixing between the buffer saline solution B present in the containment portion 14c of the filtering tip 14 and the collected water is made efficient by the presence of the filter F, which on the other hand, having only to eliminate micrometric particles P, does not alter the concentration of salts present in the water and buffer mixture in any manner.

The microfluidic measuring cartridge 20 will now be described in detail.

The cartridge 20 contains one or more sensors 26 which physically measure the concentration of electrochemically active substance being analyzed.

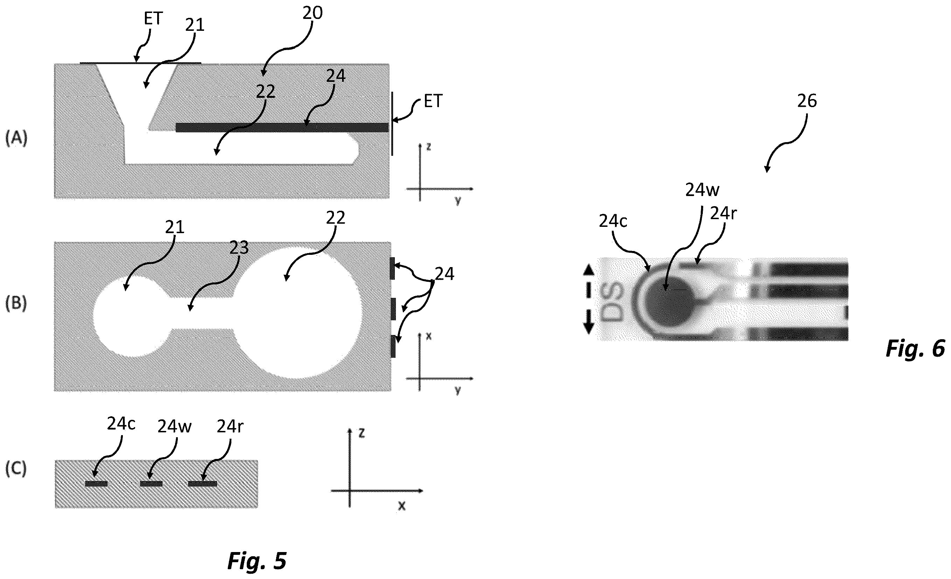

The simplified diagram of the cartridge 20 is presented in Figure 5 wherein only one set of electrodes is present.

In particular, Figure 5A shows a side section, Figure 5B shows a longitudinal section seen from above, and Figure 5C shows a front section of the cartridge 20.

The inlet of the cartridge 20 and electrical contacts are protected by a tear-off label ET similar to those which protect the ink-jet printer cartridges.

The water sample C is injected into the cartridge 20 by means of the syringe 10 equipped with the filtering tip 14.

During the injection of the water sample C, this is mixed with the buffer B present in the filtering tip 14 and the mixture passing through the introduction portion 14a, is received in a microfluidic reservoir 22, i.e., the depth of which is less than one millimeter. Access to the reservoir 22 is facilitated by a funnel inlet or recess 21 closed by said tear-off label ET and adapted to receive said introduction portion 14a.

Finally, electrodes 24 performing the electrochemical measurement of the concentration of the metals being analyzed are arranged on the upper face of the microfluidic reservoir 22.

The reservoir 22 and electrodes 24 form an electrochemical chamber.

The pressure pushing the water to enter into the reservoir 22 is directly provided by the injection syringe 10 whereby no active element is present in the cartridge 20 to create a pressure difference. Therefore, the water to be analyzed is inserted by means of the filtering tip 14 into the recess 21, passes through a connection duct 23 and arrives in the reservoir 22.

If avoiding the escape of even very small amounts of water during the operation of inserting the sample into the cartridge is required, for example if highly harmful pollutants are suspected to be present in the sample, the cartridge can contain a second reservoir for excess water which does not enter into the reservoir 22. This second reservoir is not shown in Figure 5.

To perform the electrochemical measurement of the concentration of electrochemically active substances in the water sample, three electrodes are required, referred to as the working electrode 24w, the reference electrode 24r, and the counter electrode 24c.

There are many different possibilities for both the shape of the electrodes and the material of which they are made. The shape of the electrodes affects both the sensitivity of the measurement and the constructional simplicity of the electrochemical chamber.

The most common shape for the metal electrodes, shown in Figure 6, is that including the working electrode 24w, which must be the widest and circular in shape, and the counter electrode 24c in the shape of a circular crown which wraps the working electrode 24w. In this configuration the reference electrode 24r is usually very small and is located in a space left open by the counter electrode 24c. This configuration is shown in Figure 6.

The circular configuration is the easiest to construct, especially if the support material is a polymer, but it is not the most efficient in terms of sensitivity. If increasing the sensitivity is required, interdigitated forms can be adopted, for example.

In these cases, the correct design in terms of shape and position of the different parts of the electrode is important to obtain the desired result, but it is possible to obtain, with the same total size of the electrode, a significant increase in sensitivity with respect to a simple circular design.

Very often, to increase the measurement sensitivity, the electrodes are coated with active molecules which make the electronic exchange between the ions in solution and the electrodes themselves more efficient, as described in the document to M.B. Gumpu et al., "A Review on Detection of Heavy Metal Ion in Water - an Electrochemical Approach, Sensors and Actuators B": Chemical, volume 213, pp. 515-533, 2015.

It is worth noting that the simplicity of the structure of the cartridge 20 is due in large part to the choice to incorporate the function of mixing the electrochemical buffer B and the water sample C into the filtering tip 14 of the syringe 10.

This function, i.e., the creation of the mixture, could also be inserted into the cartridge 20 itself, loading the cartridge 20 with the buffer B and implementing a microfluidic mixer therein. In this case the filtering tip 14 does not contain the buffer B therein, but only includes the filter F.

Certainly, the solution of performing the mixing in the filtering tip 14 of the syringe 10 is simpler, but it cannot be adequate if using buffers B which are harmful to the user of the system is required. In this case, in order to avoid complicating the use procedure and introducing elements of risk, the mixing can be performed in the microfluidic chip, storing the buffer therein. In this case, a microfluidic mixer must be introduced into the chip, for example, of a diffusive or chaotic type.

The reader 30 for reading from the cartridge 20 contains all the electronics required to process the analog signal from the cartridge 20 and present the data in a comprehensible manner.

The shape of the reader 30 is ergonomic, so that it can be used even in the absence of stable supports.

The cartridge 20 is inserted into the reader 30 by means of a multi-contact interface. Three contacts are needed to impose the test voltage on the electrochemical cell and to read the resulting current. Since these are small currents, the contacts must be protected from both degradation due to environmental agents and background noise.

In the case of a stand-alone reader 30A, in addition to an On/Off button and a button to start the test, the reader 30A allows entering all the data related to the measurement (date, mode, place of collection, notes, etc...) and selecting how the data must be presented.

In particular, in addition to a real-time presentation on a screen 32, storing data on a memory (e.g., an SSD card) and/or transmitting them on the network must be possible for storage in a cloud.

In the first analysis, a touch screen 32 could be adapted to carry out not only the functions of data presentation, but also those of setting and storing the measurement conditions, leaving only two physical buttons, one for On/Off and one for starting the measurement.

The reader 30A hosts a resident software package which carries out multilevel functions.

In particular, the functions carried out by the reader 30 are as follows:
- processing the analog coming data from the electrochemical sensor 26 to produce the measurement of the desired concentrations with an error estimate;
- implementing the local interface with the user to guide them in using the system as easily as possible and presenting measurement data or error messages on the local screen 32;
- managing a wireless connection to the web by means of SIM which allows recording data remotely and, when necessary, the initialization of the device and software; and
- the reader 30, once the cartridge 20 has been inserted, detects the serial number of the cartridge 20 so as to allow several different measurements with the same reader.

In the case of a reader 30B interfacing with another computing device, such as a smartphone 40, these functions are carried out by the App loaded onto the device 40.

The procedure of using a portable device must be particularly simple so as to make it possible for staff without any specialization in the field to use the device.

In particular, in this case the use procedure of use can be summarized with the following steps:
a. switching on the reader 30 by pressing the on button;
b. removing the label which protects the electrical contacts and inserting the cartridge 20 into the reader 30;
c. extracting the syringe 10 from the package (the collection tip 13 will already be assembled on the syringe 10);
d. immersing the collection tip 13 (but not the syringe 10) in water;
e. aspirating a water sample C up to the point marked on the syringe 10, this operation must be done carefully to keep the amount of water collected under control;
f. overturning the syringe 10 and removing the collection tip 13, which must be placed in the package to be then recycled;
g. assembling the filtering tip 14; during assembly, the closure label 14d of the containment portion 14c of the tip 14 containing the measurement saline solution or buffer B is broken, and the mixing between the buffer B and the water sample C begins;
h. after assembling the filtering tip 14 on the syringe 10, rotating the introduction portion 14a with respect to the containment portion 14c so as to tear the membrane M to allow the passage of the liquid from the containment portion 14c to the tip of the introduction portion 14a,
i. removing the protection label ET of the cartridge 20 thus opening the inlet thereof;
l. bringing the end opening of the filtering tip 14 in contact with the inlet of cartridge 20 at the recess 21;
m. injecting the water of the syringe 10 into the cartridge 20 (the water will not exit the cartridge 20 due to the surface tension);
n. pressing the start button on the reader 30;
o. placing the syringe 10 with the assembled filtering tip 14 in the package for later recycling;
p reading the result on the screen 32 after the set time;
q. extracting the cartridge 20;
r. switching off the reader 30, and
s. placing the used cartridge in the package for disposal or recycling.

Generally, electrochemical measuring systems must be periodically calibrated due to the changes which occur in the electrodes despite the washing and regeneration processes.

In the case in hand, the cartridges are of the single-use type. After a single measurement, the cartridge 20 is collected and recycled or regenerated by means of suitable processes. In any case it cannot be used more than once in a row.

This means that once the single cartridge has been calibrated in the factory, no calibration is required and it can simply be used without other necessary operations.

In mass production, of course, not all cartridges 20 will have the same calibration and it should be expected that especially different production batches can have different calibrations. In order to cope with this problem, it is required for each cartridge 20 to carry its calibration coefficient therewith and communicate it to the reader 30 when the cartridge 20 is inserted.

This feature can be added to the cartridge 20 by providing it with a passive, inductively-activated chip, such as those present, for example, in bank or credit cards. The reader 30, provided with a suitable receiving circuit, can read the calibration coefficient at the moment of the insertion of the cartridge 20 and then use it to interpret the raw measurement.

The procedure for constructing the cartridge 20 is now described.

The shape and size of the cartridge 20 will be defined both by the size required for the electrochemical cell to achieve the required resolution and by criteria of ergonomics and mechanical resistance.

Therefore, the total size of the cartridge 20 will probably be of the order of at least 20-30 cm² of horizontal area with a depth not less than 1.5-2 cm.

The material of which the cartridge 20 is made must be capable of stably depositing thin films of metals, graphite or similar compounds which will form the electrochemical electrodes. These films must have a highly repeatable and regular structure so as not to have excessive resistance or fluctuations in features from piece to piece.

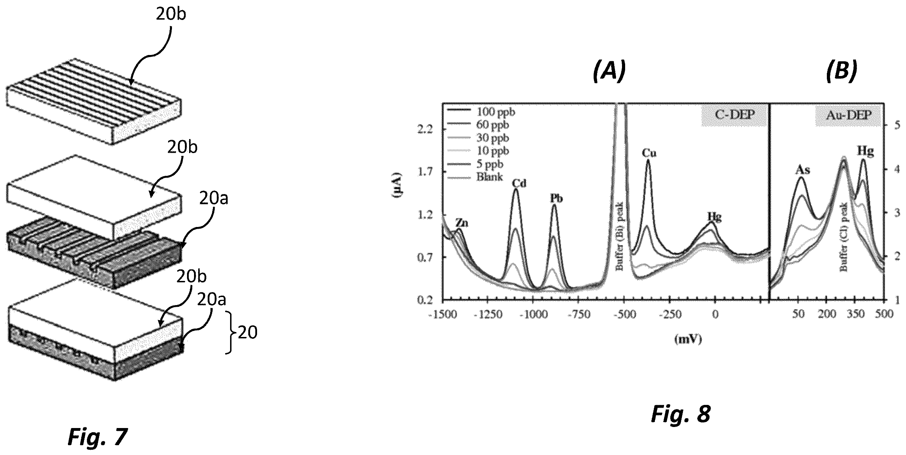

Based on these preliminary considerations, with reference to Figure 8, the best manner to construct the cartridge 20 is to construct the entire cartridge 20 as a hollow shape 20a (which forms the reservoir 22) by micromolding using high-melting polymers, for example some variant of polysulfone or Polyether ether ketone. The hollow shape 20a is then covered with a cover 20b made of a material suitable for the deposition of the electrodes 24 which can be fixed by industrial gluing given the dimensions of the cartridge 20.

The first possible choice is glass, on which the deposition processes of thin films by evaporation or sputtering are well established, but this could not be the most convenient choice. As a function of the metals or other materials which must form the electrodes, both a polymer solution and the use of different materials, such as alumina, which would allow the use of the screen-printing method, well established in electrochemistry, should certainly be investigated.

The key to the operation of the system is the electrochemical measurement of the concentration of electrochemically active substance ions in water. Metals are among the materials which give a stronger and more defined electrochemical response, as shown in Figure 9, which shows the electrochemical response of seven different metal ions in water in the typical concentrations of pollutants which must be identified in waste water or drinking water.

The measurements were performed with working electrodes of carbon (graph (A) on the left) and gold (graph (B) on the right) and show the very clear definition of the peaks in the voltage-current response corresponding to each of the metals considered and the intensity of the corresponding current, which is of the order of microamps.

In Figure 9 the voltammograms (the voltage-current curves) are obtained with the differential pulse voltammetry technique. The graph on the left (A) shows the detection of zinc, cadmium, lead, copper and mercury using a working electrode and a graphite counter electrode, while the graph on the right (B) shows the detection of arsenic and mercury using a gold working electrode. In both cases the reference electrode is an Ag/AgCI pseudo-electrode. The data are obtained from averages of four to six independent measures.

One of the manners to improve the sensitivity of the electrodes is to grow a monolayer of a suitable macromolecule on the surface thereof which interacts by means of an appropriate oxidation reaction with the substance to be measured. In the case of many metals, such as Cadmium or Mercury, these are enzymatic inhibition reactions which involve a more efficient exchange of charges with the electrode with respect to the simple reaction with the target ion.

However, this technique has several disadvantages with respect to the use of simple electrodes:
- the electrode is usually specialized to a single electrochemically active substance, whereby if measuring several substances simultaneously is required, several electrodes need to be constructed;
- the temperature resistance of the enzymes used must be established accurately;
- the stability of the single layer deposited on the electrode must be carefully measured so as not to intolerably reduce the life time of the cartridge; and
- the cost of the cartridge is higher.

An interesting alternative to the deposition of macromolecule monolayers is the modification of the surface by means of deposition of nanostructured materials. These materials have several effects which when combined increase the sensitivity and selectivity of the electrode.

The presence of a granular structure on the surface considerably increases the effective area in terms of redox of small targets, such as metal ions, effectively increasing the sensitivity of the electrode. In addition, this structure makes it difficult to react with larger interfering agents, such as organic molecules or large inorganic molecules, thus increasing the selectivity of the electrode.

Moreover, nanometer dimensions are often associated with chemical, optical, magnetic, thermal, and electronic properties which are different from those exhibited by the same material with larger dimensions, i.e., by "bulk" material, which can be utilized to construct electrochemical sensors with higher performance.

Referring to modification by means of deposition of metallic nanoparticles (NPs), many synthesis techniques have been developed, both physical and chemical, capable of producing nanoparticles of different size, shape and composition.

A first set of methods for modifying the surface by means of nanoparticles, referred to as the physical approach, includes techniques such as laser ablation, lithography and high-energy irradiation.

A second set of methods, referred to as the chemical approach, is essentially based on reduction reactions, conducted with appropriate reagents, or electrochemically or photocatalytically.

The wealth of possible methods for the synthesis of this type of electrodes makes them very interesting and versatile. Moreover, modification by means of nanoparticles lends itself to making electrodes suitable for measuring multiple metals simultaneously and is generally found to have fewer stability problems with respect to activation by means of single-layer macromolecules.

Making the system portable means avoiding an excessive number of manual actions, or complex actions despite the fact that the amounts injected into the cartridge and the concentration of salts must be controlled; in this respect, it should be noted that it is possible to perform the mixing between the water being measured and the reference saline solution also inside the cartridge by means of a microfluidic mixer, which would solve the problem of amount control; at present, however, this solution seems more expensive than integrating the operation into the syringe.

With reference to the saline concentration, it could be problematic to get this method work for water with high concentrations of salts.

The electrochemical measurement must achieve a great sensitivity while operating with small electrodes and under varying temperature conditions; for this reason, the design of the shape and structure of the electrodes is fundamental.

The electrochemical cartridge must be constructed so as to be stable with temperature and time and at low cost: this affects both the material of which the cartridge is made and the construction of the electrodes.

The current which is generated by the electrochemical cartridge could be very low, much less than the currents detected in Figure 9, which are generated with a laboratory procedure; this places strict requirements on the contact between the cartridge and the reader which however must be achieved by means of a low-cost solution.

The reader itself must have a quality analog front-end so as not to introduce excessive noise or deformation into the signal before the amplification and digitization thereof.

It should be noted that the microfluidic technology for performing operations inside the chip such as mixing the buffer with the sample and washing is a well-established technology for which the necessary microfluidic chip, although more complex than that required for the portable system, is still achievable with known and well-tested technologies.

The above description of embodiments of the invention is capable of showing the invention from a conceptual point of view so that others, using the known art, will be able to modify and/or adapt, in various applications, such specific embodiments, without further research and without departing from the inventive concept, and therefore it is understood that such adaptations and modifications will be considered as equivalents of the specific embodiments.

The means and materials for achieving the various described functions can be of various types without departing from the scope of the invention.

The expressions or terminology used are merely intended for descriptive purposes and therefore are not limiting.

Obviously, without prejudice to the principle of the invention, the construction details and the embodiments can widely vary with respect to the above disclosure merely given by way of example, without departing from the scope of the present invention.

Where the constructional features and techniques mentioned in the following claims are followed by references signs or numerals, such reference signs were introduced for the sole purpose of increasing the intelligibility of the claims themselves, and therefore they have no limiting effect on the interpretation of each element identified, by way of mere example, by such reference signs.

## Claims

1. A portable system for measuring the concentration of electrochemically active substances in water with electrochemical techniques comprises:
- a device (10,13) for collecting a sample (C) of water to be analyzed,
- a device (10,14) adapted to mix the collected water sample (C) with a saline solution (B),
- a measuring cartridge (20) for analyzing the sample and saline solution mixture, wherein said cartridge is provided with at least one sensor (24),
- a reader (30) adapted to read the data produced by the sensor (24) of said measuring cartridge (20), and
- a data processing unit provided with a user interface for communication with an operator and a network interface for communication with a remote server or other type of remote apparatus acting as a user of the information, wherein said data processing unit converts the data from the analog form in which they are produced by the sensor (24) of the cartridge (20) into a digital format suitable for being further processed.

2. The portable system for measuring the concentration of electrochemically active substances in water with electrochemical techniques according to claim 1, wherein the device (10,13) for collecting a water sample (C) is a syringe (10) comprising a main containment body (11) provided with a plunger (11b) and a connection spout (12) for the coupling and connection with a collection tip (13) and a filtering tip (14).

3. The portable system for measuring the concentration of electrochemically active substances in water with electrochemical techniques according to claim 3, wherein the collection tip (13) comprises an aspiration portion (13a) and a connection element (13b), wherein the aspiration portion (13a) is hollow and allows the passage of liquids and is curved to facilitate the operation of extracting the sample (C).

4. The portable system for measuring the concentration of electrochemically active substances in water with electrochemical techniques according to claim 3, wherein the device (10,14) adapted to mix the collected water sample (C) with a saline solution (B) is included in the filtering tip (14).

5. The portable system for measuring the concentration of electrochemically active substances in water with electrochemical techniques according to claim 4, wherein the filtering tip (14) comprises an introduction portion (14a), a connection element (14b), and a containment portion (14c) containing said saline solution (B), and wherein the connection element (14b) is sealed by a breaking closure (14d).

6. The portable system for measuring the concentration of electrochemically active substances in water with electrochemical techniques according to claim 4, wherein the containment portion (14c) and the introduction portion (14a) in the filtering tip (14) are two distinct parts inserted into each other so that they can rotate with respect to each other, wherein a filtering portion (F) is provided between the containment portion (14c) and the introduction portion (14a), and wherein the containment portion (14c) is sealed by a membrane (M).

7. The portable system for measuring the concentration of electrochemically active substances in water with electrochemical techniques according to any one of the preceding claims 2-6, wherein the connection between the connection spout (12) and the connection element (13b, 14b) of the collection tip (13) or of the filtering tip (14) occurs by interlocking or screwing to avoid accidental removal.

8. The portable system for measuring the concentration of electrochemically active substances in water with electrochemical techniques according to any one of the preceding claims, wherein said cartridge is a microfluidic measuring cartridge (20).

9. The portable system for measuring the concentration of electrochemically active substances in water with electrochemical techniques according to claim 8, wherein said microfluidic measuring cartridge (20) contains at least one sensor (26) which physically performs the measurement of the concentration of electrochemically active substances.

10. The portable system for measuring the concentration of electrochemically active substances in water with electrochemical techniques according to claim 9, wherein said cartridge (20) comprises a funnel inlet or recess (21) which through a connection duct (23) is connected to a microfluidic reservoir (22), and wherein electrodes (24) creating said sensor (26) are arranged on the upper face of the microfluidic reservoir (22).

11. The portable system for measuring the concentration of electrochemically active substances in water with electrochemical techniques according to claim 10, wherein said sensor (26) comprises a working electrode (24w), a reference electrode (24r), and a counter electrode (24c).

12. The portable system for measuring the concentration of electrochemically active substances in water with electrochemical techniques according to any one of the preceding claims 8-11, wherein the inlet of the cartridge (20) and the electrical contacts (24w,24r,24c) of the sensor (26) are protected by a tear-off label (ET).

13. The portable system for measuring the concentration of electrochemically active substances in water with electrochemical techniques according to any one of the preceding claims 1-3, wherein the device (10,14) adapted to mix the collected water sample (C) with a saline solution (B) is a microfluidic mixer of the diffusive or chaotic type included in the measuring cartridge (20).

14. The portable system for measuring the concentration of electrochemically active substances in water with electrochemical techniques according to any one of the preceding claims, wherein the reader (30A) for reading from the cartridge (20) contains all the electronics required to process the analog signal from the cartridge (20) and present the data in a comprehensible manner.

15. The portable system for measuring the concentration of electrochemically active substances in water with electrochemical techniques according to any one of the preceding claims 1-12, wherein the reader (30B) for reading from the cartridge (20) utilizes the electronics of a smart device (40) to process the analog signal from the cartridge (20) and for the interaction of the reader (30B) with the operator.

16. A method for measuring the concentration of electrochemically active substances in water with electrochemical techniques comprising the steps of providing a measuring system according to one or more of claims 1 to 14.

17. The method for measuring the concentration of electrochemically active substances in water with electrochemical techniques according to claim 15, comprising the steps of:
a. switching on the reader (30);
b. removing the label (ET) which protects the electrical contacts and inserting the cartridge (20) into the reader (30);
c. extracting the syringe (10) from the package with the collection tip (13) already assembled on the syringe (10);
d. immersing the collection tip (13) in water;
e. aspirating a sample (C) of water up to the point marked on the syringe (10);
f. overturning the syringe (10) and removing the collection tip (13);
g. assembling the filtering tip (14); during assembly, the closure label (14d) of the containment portion (14c) containing the measurement saline solution (B) is broken, and the mixing between the buffer (B) and the water sample (C) begins;
h. after assembling the filtering tip (14) on the syringe (10), rotating the introduction portion (14a) with respect to the containment portion (14c) so as to tear the membrane (M) to allow the passage of the liquid from the containment portion (14c) to the tip of the introduction portion (14a),
i. removing the protection label (ET) of the cartridge (20) thus opening the inlet thereof;
l. bringing the end opening of the filtering tip (14) in contact with the inlet of cartridge (20) at the recess (21);
m. injecting the water of the syringe (10) into the cartridge (20);
n. pressing the start button on the reader (30);
o. placing the syringe (10) with the assembled filtering tip (14) in the package for later recycling;
p reading the result on the screen (32) after the set time;
q. extracting the cartridge (20);
r. switching off the reader (30); and
s. placing the used cartridge (20) in the package for disposal or recycling.

18. The method for measuring the concentration of electrochemically active substances in water with electrochemical techniques according to claim 15, comprising the steps of:
a. switching on the reader (30);
b. removing the label (ET) which protects the electrical contacts and inserting the cartridge (20) into the reader (30);
c. extracting the syringe (10) from the package with the collection tip (13) already assembled on the syringe (10);
d. immersing the collection tip (13) in water;
e. aspirating a sample (C) of water up to the point marked on the syringe (10);
f. removing the protection label (ET) of the cartridge (20) thus opening the inlet thereof;
g. bringing the end opening of the filtering tip (14) in contact with the inlet of cartridge (20) at the recess (21);
h. injecting the water of the syringe (10) into the cartridge (20) to activate the microfluidic mixer of diffusive or chaotic type included in the microfluidic measuring cartridge (20) to mix the measurement saline solution (B) with the water sample (C);
i. pressing the start button on the reader (30);
l. placing the syringe (10) in the package for later recycling;
m. reading the result on the screen (32) after the set time;
n. extracting the cartridge (20);
o. switching off the reader (30); and
p. placing the used cartridge (20) in the package for disposal or recycling.
